# EUROPÄISCHE PATENTSCHRIFT

(11) **EP 1 685 813 B1**
(45) Veröffentlichungstag und Bekanntmachung des Hinweises auf die Patenterteilung: **05.08.2009**
(21) Anmeldenummer: 06001450.3
(22) Anmeldetag: 24.01.2006
(51) Int. Cl.: A61F 2/88, A61F 2/06, A61M 25/00

(54) **Stent-graft**
Stent-graft
Stent greffe

(30) Priorität: 28.01.2005 DE 202005001416 U
(43) Veröffentlichungstag der Anmeldung: 02.08.2006
(73) Patentinhaber: Urovision Gesellschaft für Medizinischen Technologie Transfer mbH, 83043 Bad Aibling (DE)
(72) Erfinder: Berg, Hendrik, 83122 Samerberg - Törwang (DE); Maier, Florian, 83043 Bad Aibling (DE)
(74) Vertreter: Patentanwälte Hofstetter, Schurack & Skora

(56) Entgegenhaltungen:
- EP-A- 1 330 993
- WO-A-00/49973
- WO-A-20/05086942
- DE-A1- 10 235 868
- US-A- 4 300 244
- US-A- 5 151 105
- US-A1- 2004 019 375
- US-A1- 2004 181 186
- US-B1- 6 699 277

## Beschreibung

Die vorliegende Erfindung betrifft einen Stent zum Einführen und Einsetzen in menschliche oder tierische Körperhöhlen mit einem distalen Einführende und einem proximalen Ende sowie einem Stentkörper mit einem Lumen, wobei das Lumen einen vordefinierten Durchmesser aufweist und der Stentkörper aus mindestens einem elastischen, biokompatiblen Kunststoffmaterial besteht und am Innenumfang des Stentkörpers im Bereich zwischen dem proximalen Ende und dem distalen Einführende zumindest teilweise mindestens ein metallisches Stützgerüst angeordnet ist.

Derartige Stents sind aus der US-A-5 151 105 und der US-A-4 300 244 bekannt. Dabei werden Stents insbesondere als Gefäßprothesen zur Abstützung von Gefäßinnenwänden benutzt. Bekannte Stents weisen üblicherweise ein röhrenförmiges Stützgerüst aus Metall auf, wobei bei dem Einführen der Stents in die entsprechenden Körperhöhlen diese zusammengedrückt werden. Ist der Stent dann an der vorbestimmten Gefäßendstelle platziert, so weitet sich das Stützgerüst derart, dass es einen größeren Durchmesser als im Einführzustand aufweist. Das Aufweiten kann dabei mit Hilfe entsprechender Vorrichtungen oder selbsttätig erfolgen. Weitere Beispiele für derartige Stents sind aus der DE 91 16 936 U1, der DE 695 10 973 T2, der DE 694 31 090 T2, der US2003/ 00 40 803 A1, der US-A-5 540 701 A, der WO 04/0 41 345 A1, der DE 202 20 419 U1, der DE 699 08 736 T2 und der DE 697 27 617 T2 bekannt.

Nachteilig an den bekannten Stents ist jedoch, dass bei dem Einführen in die menschlichen oder tierischen Körperhöhlen immer wieder Probleme durch die sehr geringe oder nicht vorhandene Längsdehnung der bekannten Metallstents auftreten. Zudem kommt es bei einem längeren Verbleib des metallenen Stents in der Körperhöhle immer wieder zu Verwachsungen zwischen dem umgebenden Gewebe und dem Stent. Eine nachträgliche Entfernung des Stents ist daher nur sehr schwer möglich und erfordert üblicherweise einen zusätzlichen operativen Eingriff, der eine entsprechende Belastung für den Patienten darstellt. Des Weiteren können zusätzliche Elemente an der Außenseite der Stents deren Einführen in Körperhöhlen erschweren und deren Elastizität verringern.

Es ist daher Aufgabe der vorliegenden Erfindung, einen gattungsgemäßen Stent bereitzustellen, welcher sich einerseits leicht in die Körperhöhle einführen lässt und andererseits eine Stützfunktion des umgebenden Gewebes der Körperhöhle erfüllt, ohne das es zu Verwachsungen zwischen dem Gewebe und dem Stent kommt.

Zur Lösung dieser Aufgabe dient ein Stent gemäß den Merkmalen des Patentanspruchs 1.

Vorteilhafte Ausgestaltungen des erfindungsgemäßen Stents sind in den Unteransprüchen beschrieben.

Ein erfindungsgemäßer Stent zum Einführen und Einsetzen in menschliche oder tierische Körperhöhlen weist ein distales Einführende und ein proximales Ende sowie einen Stentkörper mit einem Lumen auf, wobei das Lumen einen vordefinierten Durchmesser aufweist und der Stentkörper aus mindestens einem elastischen, biokompatiblen Kunststoffmaterial besteht, wobei am Innenumfang des Stentkörpers im Bereich zwischen dem proximalen Ende und dem distalen Einführende - jeweils beabstandet von den Enden - zumindest teilweise mindestens ein metallisches Stützgerüst angeordnet ist. Erfindungsgemäß sind zur Fixierung des mindestens einen metallischen Stützgerüstes nur dessen jeweilige Enden zwischen sich verjüngenden Bereichen des Stentkörpers am Innenumfang des Stentkörpers zu liegen kommen, wobei die verjüngenden Bereiche durch eine Verringerung des Innen- und Außendurchmessers des Stentkörpers ausgebildet sind. Eine weitere Möglichkeit zur Fixierung des mindestens einen metallischen Stützgerüsts besteht darin, dass zumindest dessen jeweilige Enden mit dem Innenumfang des Stentkörpers verschweißt sind. Dabei kann in die Enden zumindest teilweise Material des Stentkörpers eingeschmolzen werden. Es ist aber auch möglich, dass zur Fixierung des metallischen Stützgerüsts dessen jeweilige Enden mit dem Innenumfang des Stentkörpers verklebt sind. Die vorgenannten Fixierungsmöglichkeiten gewährleisten alle einen sicheren Halt des mindestens einen metallischen Stützkörpers innerhalb des Stentkörpers, ohne dass dessen Elastizität und Flexibilität, insbesondere in der Längserstreckung, wesentlich beeinträchtigt wird. Durch eine derartige Ausführung des erfindungsgemäßen Stents wird zudem zuverlässig verhindert, dass es zu Verwachsungen zwischen dem Gewebe und dem Stent kommt, da das metallische Stützgerüst nicht in Berührung zu dem umgebenden Gewebe kommt. Das Kunststoffmaterial verhindert Verwachsungen zuverlässig. Des Weiteren erlaubt die erfindungsgemäße Ausgestaltung des Stents ein leichtes Einführen und Einsetzen des Stents, da einerseits das elastische Kunststoffmaterial eine gute Gleitfähigkeit aufweist und so zu einer leichten Einführung in die entsprechenden Körperhöhlen beiträgt. Zudem sind keine zusätzlichen Elemente an der Außenseite der Stents angeordnet. Des Weiteren sind die beiden Enden des Stentkörpers nicht mit dem metallischen Stützgerüst ausgekleidet, so dass diese flexibel und elastisch und damit leicht einführbar sind. Des Weiteren ergibt sich eine im Vergleich zu üblichen Stents deutlich erhöhte Längsausdehnung des Stents insgesamt, was wiederum zu einem erleichterten Einführen in die entsprechenden Körperhöhlen beiträgt. Schließlich lassen sich erfindungsgemäße Stents einfacher und damit kostengünstiger herstellen. Insbesondere kann für die Herstellung des Stentkörpers ein einziges Material verwendet werden, was zudem Vorteile bei der Haltbarkeit des erfindungsgemäßen Stents gegenüber bekannten, aus zwei oder mehreren Materialen bestehenden Stentkörpern bzw. Stents aufweist.

In einer vorteilhaften Ausgestaltung der Erfindung besteht das Stützgerüst aus mindestens einer umlaufenden, unterbrochenen und/oder nicht-unterbrochenen Spiralfeder. Eine derartige Ausgestaltung des metallischen Stützgerüsts verstärkt vorteilhafterweise den Dehneffekt in Längsrichtung des Stents. Dies wiederum trägt zu einem leichteren Ein- und Ausführen des Stents aus der Körperhöhle bei. Es ist aber auch möglich, dass das Stützgerüst aus mindestens einem umlaufenden, unterbrochenen und/oder nicht-unterbrochenen Ringelement besteht. Auch hierdurch ergibt sich ein vorteilhafter Dehneffekt in Längsrichtung des Stents.

In einer weiteren vorteilhaften Ausgestaltung der Erfindung sind der Stentkörper und das metallische Stützgerüst in Richtung senkrecht zur Längsachse des Stents nicht-expandierend ausgebildet. Eine derartige Ausgestaltung ermöglicht es, den Stent kostengünstig herstellen zu können, da auf aufwändige Werkzeuge zur Expandierung des Stents innerhalb der Körperhöhle oder auf sehr teure selbstexpandierende Materialien, wie zum Beispiel Gedächtnismetalle und -legierungen verzichtet werden kann. So kann das metallische Stützgerüst beispielsweise aus medizinischem Edelstahl bestehen. Dabei kann ein Flach- oder Runddraht verwendet werden. Der Stentkörper selbst besteht üblicherweise aus Polyurethan.

In einer weiteren vorteilhaften Ausgestaltung der Erfindung weisen das proximale Ende und/oder das distale Einführende und/oder der Stentkörper Drainageöffnungen auf. Dadurch ist es möglich, den erfindungsgemäßen Stent als Drainagekatheter, Pigtail-Katheter, als Dauerkatheter oder auch als Ballonkatheter sowie als Uretherschiene zu verwenden.

Weitere Einzelheiten, Merkmale und Vorteile der Erfindung ergeben sich aus dem in den Figuren dargestellten Ausführungsbeispiel. Es zeigen
- Figur 1: eine schematische Darstellung des erfindungsgemäßen Stents; und
- Figur 2: eine schematische Schnittdarstellung eines Teilabschnitts des erfindungsgemäßen Stents.

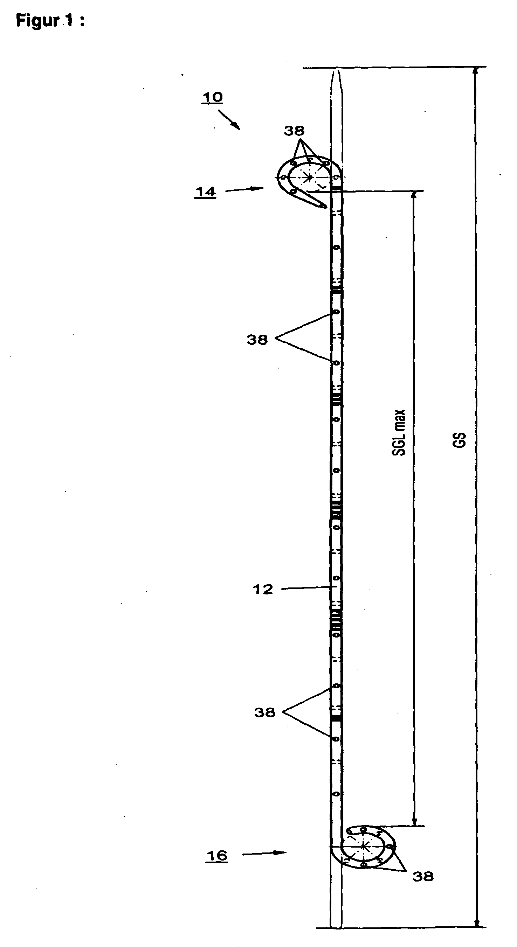

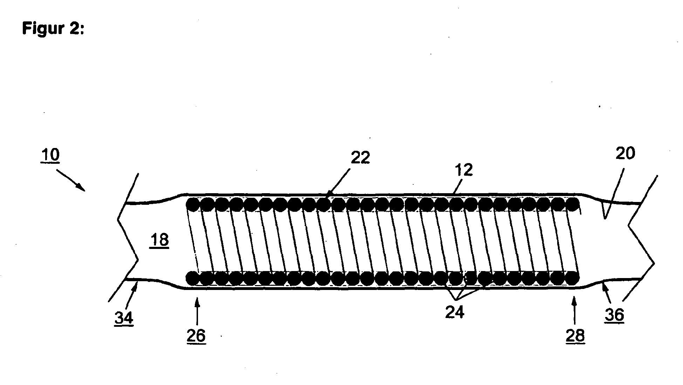

Figur 1 zeigt in einer schematischen Darstellung einen Stent 10 zum Einführen und Einsetzen in menschliche oder tierische Körperhöhlen mit einem distalen Einführende 14 und einem proximalen Ende 16 sowie einem Stentkörper 12 mit einem Lumen 18 (vergleiche Figuren 2 bis 4). Der Stentkörper 12 besteht dabei aus einem elastischen, biokompatiblen Kunststoffmaterial, insbesondere aus Polyurethan. Zudem besteht die Möglichkeit, dass der Stentkörper 12 eine weitere Beschichtung zur Verbesserung der Gleitfähigkeit aufweist. Bei dem dargestellten Stent handelt es sich um einen sogenannten Pigtail-Katheter, der zusätzlich eine Stentfunktion ausübt. Man erkennt, dass an dem proximalen und distalen Ende 16, 14 sowie im Stentkörper Drainageöffnungen 38 ausgebildet sind. Des Weiteren ist in der Figur 1 die maximale Länge SGL max eines metallischen Stützgerüsts 22 (vergleiche auch Figuren 2 bis 4) angedeutet, wobei das metallische Stützgerüst 22 am Innenumfang 20 des Stentkörpers 12 im Bereich zwischen dem proximalen Ende 16 und dem distalen Einführende 14 angeordnet ist. Zudem ist die gestreckte Länge GS des Stents 10 dargestellt. Durch die Anordnung des metallischen Stützgerüsts zwischen den Enden 14, 16 und am Innenumfang 20 des Stentkörpers 12 wird die maximale Streckung des Stents 10 in Längsrichtung, d. h. parallel zur Längsachse des Stents 10, nur unwesentlich oder nicht beeinflusst, so dass der Stent 10 durch seine Elastizität insgesamt insbesondere an den Ende 14, 16 sehr leicht in die entsprechende menschliche oder tierische Körperhöhle ein- und ausführbar ist.

Figur 2 zeigt eine schematische Schnittdarstellung eines Teilabschnitts des Stents 10. Das metallische Stützgerüst 22 besteht aus einer umlaufenden Spiralfeder 24. Zur Fixierung der Spiralfeder 24 kommen die Enden 26, 28 des Stützgerüsts 22 zwischen sich verjüngenden Bereichen 34, 36 des Stentkörpers 12 zu liegen. In dem dargestellten Ausführungsbeispiel wird dies durch eine Verringerung des Innendurchmessers des Stentkörpers 12 erreicht. Das metallische Stützgerüst 22 besteht aus medizinischem Edelstahl, wobei zur Herstellung des Stützgerüsts 22 Flach- bzw. Runddraht verwendet werden kann. Es ist aber auch möglich, dass das Stützgerüst aus mindestens einem umlaufenden, unterbrochenen und/oder nicht-unterbrochenen Ringelement besteht (nicht dargestellt).

## Patentansprüche

1. Stent zum Einführen und Einsetzen in menschliche oder tierische Körperhöhlen mit einem distalen Einführende (14) und einem proximalen Ende (16) sowie einem Stentkörper (12) mit einem Lumen (18), wobei das Lumen (18) einen vordefinierten Durchmesser aufweist und der Stentkörper (12) aus mindestens einem elastischen, biokompatiblen Kunststoffmaterial besteht und am Innenumfang (20) des Stentkörpers (12) im Bereich zwischen dem proximalen Ende (16) und dem distalen Einführende (14) zumindest teilweise mindestens ein metallisches Stützgerüst (22) angeordnet ist,
**dadurch gekennzeichnet,**
**dass** zur Fixierung des mindestens einen metallischen Stützgerüstes (22) nur dessen jeweilige Enden (26, 28) zwischen sich verjüngenden Bereichen (34, 36) des Stentkörpers (12) am Innenumfang (20) des Stentkörpers (12) zu liegen kommen, wobei die verjüngenden Bereiche (34, 36) durch eine Verringerung des Innen- und Außendurchmessers des Stentkörpers (12) ausgebildet sind.

2. Stent nach Anspruch 1,
**dadurch gekennzeichnet,**
**dass** das Stützgerüst (22) aus mindestens einer umlaufenden, unterbrochenen und/oder nicht-unterbrochenen Spiralfeder (24) besteht.

3. Stent nach Anspruch 1,
**dadurch gekennzeichnet,**
**dass** das Stützgerüst aus mindestens einem umlaufenden, unterbrochenen und/oder nicht-unterbrochenen Ringelement besteht.

4. Stent nach einem der vorhergehenden Ansprüche,
**dadurch gekennzeichnet,**
**dass** der Stentkörper (12) und das metallische Stützgerüst (22) in Richtung senkrecht zur Längsachse des Stents (10) nicht-expandierend ausgebildet sind.

5. Stent nach einem der vorhergehenden Ansprüche,
**dadurch gekennzeichnet,**
**dass** metallische Stützgerüst (22) aus medizinischem Edelstahl besteht.

6. Stent nach einem der vorhergehenden Ansprüche,
**dadurch gekennzeichnet,**
**dass** metallische Stützgerüst (22) aus einem Flach- oder Runddraht besteht.

7. Stent nach einem der vorhergehenden Ansprüche,
**dadurch gekennzeichnet,**
**dass** der Stentkörper (12) aus Polyurethan besteht.

8. Stent nach einem der vorhergehenden Ansprüche,
**dadurch gekennzeichnet,**
**dass** zur zusätzlichen Fixierung des mindestens einen metallischen Stützgerüstes (22) dessen jeweilige Enden (26, 28) mit dem Innenumfang (20) des Stentkörpers (12) verschweißt sind.

9. Stent nach Anspruch 8,
**dadurch gekennzeichnet,**
**dass** in die Enden (26, 28) zumindest teilweise Material (40) des Stentkörpers (12) eingeschmolzen ist.

10. Stent nach einem der vorhergehenden Ansprüche,
**dadurch gekennzeichnet,**
**dass** zur zusätzlichen Fixierung des mindestens einen metallischen Stützgerüstes (22) zumindest dessen jeweilige Enden (26, 28) mit dem Innenumfang (20) des Stentkörpers (12) verklebt sind.

11. Stent nach einem der vorhergehenden Ansprüche,
**dadurch gekennzeichnet,**
**dass** das proximale Ende (16) und/oder das distale Einführende (14) und/oder der Stentkörper (12) Drainageöffnungen (38) aufweisen.

## Claims

1. Stent for introducing and inserting into human or animal body cavities, including a distal introduction end (14) and a proximal end (16) as well as a stent body (12) with a lumen (18), wherein the lumen (18) has a predefined diameter, and the stent body (12) is made of at least one resilient, biocompatible plastic material, and at least one metallic support frame (22) is at least partially disposed at the inner circumference (20) of the stent body (12) in the region between the proximal end (16) and the distal introduction end (14),
**characterized in that**
for fixing the at least one metallic support frame (22), only the respective ends (26, 28) thereof come to lie at the inner circumference (20) of the stent body (12) between tapering regions (34, 36) of the stent body (12), wherein the tapering regions (34, 36) are formed by a reduction of the inner and outer diameter of the stent body (12).

2. Stent according to claim 1,
**characterized in that**
the support frame (22) is comprised of at least one circumferential, interrupted and/or non-interrupted coil spring (24).

3. Stent according to claim 1,
**characterized in that**
the support frame is comprised of at least one circumferential, interrupted and/or non-interrupted ring member.

4. Stent according to anyone of the preceding claims,
**characterized in that**
the stent body (12) and the metallic support frame (22) are formed non-expanding in the direction perpendicular to the longitudinal axis of the stent (10).

5. Stent according to anyone of the preceding claims,
**characterized in that**
the metallic support frame (22) is made of medical stainless steel.

6. Stent according to anyone of the preceding claims,
**characterized in that**
the metallic support frame (22) is comprised of a flat or round wire.

7. Stent according to anyone of the preceding claims,
**characterized in that**
the stent body (12) is made of polyurethane.

8. Stent according to anyone of the preceding claims,
**characterized in that**
for additionally fixing the at least one metallic support frame (22), the respective ends (26, 28) thereof are welded to the inner circumference (20) of the stent body (12).

9. Stent according to claim 8,
**characterized in that**
material (40) of the stent body (12) is at least partially molten into the ends (26, 28).

10. Stent according to anyone of the preceding claims,
**characterized in that**
for additionally fixing the at least one metallic support frame (22), at least the respective ends (26, 28) thereof are adhered to the inner circumference (20) of the stent body (12).

11. Stent according to anyone of the preceding claims,
**characterized in that**
the proximal end (16) and/or the distal introduction end (14) and/or the stent body (12) have draining openings (38).

## Revendications

1. Endoprothèse vasculaire destinée à être introduite et implantée dans des cavités corporelles humaines ou animales, avec une extrémité d'introduction distale (14) et une extrémité proximale (16), ainsi qu'un corps (12) d'endoprothèse vasculaire avec une lumière (18), la lumière (18) présentant un diamètre prédéfini et le corps (12) de l'endoprothèse vasculaire se composant d'au moins un matériau plastique élastique, biocompatible et au moins une structure métallique de support (22) étant disposée au moins partiellement à la périphérie intérieure (20) du corps (12) de l'endoprothèse vasculaire, dans la zone entre l'extrémité proximale (16) et l'extrémité d'introduction distale (14),
**caractérisée en ce**
**qu'**en vue de l'immobilisation de la au moins une structure métallique de support (22), seules ses extrémités respectives (26, 28) se situent entre les zones qui s'amincissent (34, 36) du corps (12) de l'endoprothèse vasculaire, à la périphérie intérieure (20) du corps (12) de l'endoprothèse vasculaire, les zones qui s'amincissent (34, 36) étant formées par le biais d'une diminution des diamètres intérieur et extérieur du corps (12) de l'endoprothèse vasculaire.

2. Endoprothèse vasculaire selon la revendication 1,
**caractérisée en ce**
**que** la structure de support (22) se compose d'au moins un ressort en spirale (24) rotatif, interrompu et/ou non interrompu.

3. Endoprothèse vasculaire selon la revendication 1,
**caractérisée en ce**
**que** la structure de support se compose d'au moins un élément annulaire rotatif, interrompu et/ou non interrompu.

4. Endoprothèse vasculaire selon l'une des revendications précédentes,
**caractérisée en ce**
**que** le corps (12) de l'endoprothèse vasculaire et la structure métallique de support (22) sont formés de manière non en expansion dans le sens vertical par rapport à l'axe longitudinal de l'endoprothèse vasculaire (10).

5. Endoprothèse vasculaire selon l'une des revendications précédentes,
**caractérisée en ce**
**que** la structure métallique de support (22) se compose d'un acier spécial médical.

6. Endoprothèse vasculaire selon l'une des revendications précédentes,
**caractérisée en ce**
**que** la structure métallique de support (22) se compose d'un fil plat ou rond.

7. Endoprothèse vasculaire selon l'une des revendications précédentes,
**caractérisée en ce**
**que** le corps (12) de l'endoprothèse vasculaire se compose de polyuréthane.

8. Endoprothèse vasculaire selon l'une des revendications précédentes,
**caractérisée en ce**
**qu'**en vue d'une immobilisation supplémentaire de la au moins structure métallique de support (22), ses extrémités respectives (26, 28) sont soudées à la périphérie intérieure (20) du corps (12) de l'endoprothèse vasculaire.

9. Endoprothèse vasculaire selon la revendication 8,
**caractérisée en ce**
**que** du matériau (40) du corps (12) de l'endoprothèse vasculaire est fondu au moins partiellement dans les extrémités (26, 28).

10. Endoprothèse vasculaire selon l'une des revendications précédentes,
**caractérisée en ce**
**qu'**en vue d'une immobilisation supplémentaire de la au moins structure métallique de support (22), au moins ses extrémités respectives (26, 28) sont collées à la périphérie intérieure (20) du corps (12) de l'endoprothèse vasculaire.

11. Endoprothèse vasculaire selon l'une des revendications précédentes,
**caractérisée en ce**
**que** l'extrémité proximale (16) et/ou l'extrémité d'introduction distale (14) et/ou le corps (12) de l'endoprothèse vasculaire présentent des ouvertures de drainage (38).
